# EUROPÄISCHE PATENTSCHRIFT

(11) **EP 2 300 642 B1**
(45) Veröffentlichungstag und Bekanntmachung des Hinweises auf die Patenterteilung: **25.07.2012**
(21) Anmeldenummer: 09757427.1
(22) Anmeldetag: 27.05.2009
(51) Int. Cl.: A61L 27/30, A61L 27/32, A61L 27/58, C25D 9/06

(54) **ELEKTROCHEMISCHES TAUCHVERFAHREN IN EINEM WÄSSRIGEN ELEKTROLYT ZUR ERZEUGUNG EINER BIOLOGISCH DEGRADATIONSSTABILEN OBERFLÄCHENSCHICHT AUF GRUNDKÖRPERN AUS TITAN ODER TITANBASISLEGIERUNGEN**
ELECTROCHEMICAL IMMERSION METHOD IN AN AQUEOUS ELECTROLYTE FOR PRODUCING A BIOLOGICALLY DEGRADATION STABLE SURFACE LAYER ON BASE BODIES MADE OF TITANIUM OR TITANIUM BASED ALLOYS
PROCÉDÉ D'IMMERSION ÉLECTROCHIMIQUE DANS UN ÉLECTROLYTE AQUEUX POUR PRODUIRE UNE COUCHE DE SURFACE BIOLOGIQUEMENT STABLE À LA DÉGRADATION, SUR DES CORPS DE BASE EN TITANE OU EN ALLIAGES À BASE DE TITANE

(30) Priorität: 03.06.2008 DE 102008026558
(43) Veröffentlichungstag der Anmeldung: 30.03.2011
(73) Patentinhaber: Königsee Implantate Und Instrumente Zur Osteosynthese GMBH, 07426 Allendorf OT Aschau (DE)
(72) Erfinder: FINGER, Ulrich, 07318 Saalfeld (DE); SCHMIDT, Jürgen, 07745 Jena (DE); SCHRADER, Christian, 38321 Denkte (DE); ORSCHLER, Frank, 07426 Königsee (DE); HÜPPNER, Andrea, 07422 Bad Blankenburg (DE)
(74) Vertreter: Meissner, Bolte & Partner
(86) Internationale Anmeldenummer: PCT/EP2009/056479
(87) Internationale Veröffentlichungsnummer: WO 2009/147045

(56) Entgegenhaltungen:
- WO-A-98/13539
- US-A- 5 478 237
- US-A1- 2008 011 613

## Beschreibung

Die Erfindung betrifft das Verfahren zur Herstellung einer modifizierbaren, optimierbaren und dabei gleichzeitig degradationsstabilen Oberflächenschicht auf Titan und Titanbasislegierungen für Implantate zur verbesserten Osteosynthese.

Die operative Versorgung von Knochenbrüchen wird im klinischen Alltag durch eine Vielzahl von Entscheidungen der behandelnden Ärzte beeinflusst. So spielen bei der Therapiewahl neben der Reduzierung von Risiken und Komplikationen der konservativen Therapie (z. B. Immobilisationsschäden) die Erwartung des Patienten (z. B. frühe Mobilisierung und Belastbarkeit) und die Weiterentwicklung der Implantate und Operationstechniken eine wichtige Rolle. Die Frage, welches Implantat zur operativen Versorgung verwendet werden soll, ist oftmals entscheidend für den Verlauf der Frakturheilung. Die Auswahl des geeigneten Implantates hängt dabei von zahlreichen patientenbezogenen Faktoren ab, wie Allgemeinzustand (Alter, Begleiterkrankungen, perioperatives Risiko) und Erwartung (Beruf, sportliche Ambitionen). Darüber hinaus werden die therapeutischen Entscheidungen von einigen unfallbedingten

Faktoren beeinflusst. Ein weiteres wichtiges Kriterium ist der Umgang mit Implantatkomplikationen. Im Idealfall kann z. B. mit einer postoperativen Reduzierung durch die Verwendung weiterentwickelter Implantate bzw. optimierter Implantatbeschichtungen auf höchstem technologischen Stand einer hohen Komplikationsrate begegnet werden. Die Weiterentwicklung der Osteosyntheseimplantate, mittlerweile oft aus Titan hergestellt, mit flachem Profil und anatomischer Formgebung verursachen bei vielen Patienten kaum Beschwerden. Dennoch können implantatbezogene Komplikationen auftreten, die eine Metallentfernung indizieren. Die Implantatdislokation, die Fehllage eines Implantates, das die Frakturheilung gefährdet oder offensichtlich einen Schaden für benachbarte Gelenke oder Weichteilstrukturen erwarten lässt, bedarf dringend der frühzeitigen Korrektur. Ebenso sind sekundäre Frakturdislokation, Implantatbruch, Knochenheilungsstörungen oder Implantatinfektionen ausreichende Indikatoren für eine Metallentfernung. Auch nach komplikationsfreier Abheilung der Fraktur, wenn das Osteosynthesmaterial seine Funktion erfüllt hat, kann es grundsätzlich entfernt werden.

Der Eigenschaftsbereich von Implantaten reicht also mittlerweile von einstellbar komplikationsloser Entfernbarkeit bis zu einstellbar Knochenwachstum unterstützende Funktion zur Beschleunigung der Frakturheilung. Titan und Titanbasislegierungen sind dafür geeignete und langjährig erprobte Implantatgrundwerkstoffe. Durch gezieltes Design der Oberflächenbeschichtung kann die Biokompatibilität anwendungsnah und patientenfreundlich weiter erhöht werden.

### Stand der Technik

Es ist bekannt, dass die Eigenschaften metallischer Implantate wie beispielsweise die Korrosionsbeständigkeit und das Zelladhäsionsvermögen sowie das Einwachsverhalten durch die Modifizierung der oberflächennahen Schicht wesentlich verbessert werden können. Diese Eigenschaftsverbesserung kann dabei sowohl durch eine geeignete Elementverteilung als auch durch eine entsprechende Morphologie der Oberflächenschicht erreicht werden. Eine wesentliche Voraussetzung für den medizinischen Einsatz besteht weiterhin in einer guten Haftfestigkeit auf dem metallischen Substrat. Dem Stand der Technik entsprechende Oberflächenschichten bestehen z. B. aus Calciumphosphaten, da die Ausbildung eines festen Verbundes zwischen Implantatoberfläche und umliegendem Gewebe durch eine gezielte, knochenähnliche Elementverteilungen gefördert wird, also Bioaktivität vorliegt. Die Funktionalisierung von Implantatmaterialien mit Calciumphosphatbeschichtungen ist auf vielen synthetischen Wegen möglich. Als bekannte Verfahren gelten dabei das Flammen - und Plasmaspritzen mit ihren hohen technologischen Anforderungen und den Nachteilen nur mangelhaft reproduzierbare, wenig haftfeste und inhomogene Schichten generieren zu können. Sowohl in technologischer als auch in qualitativer Hinsicht stellt sich die elektrochemische Variante biokompatible Schichten zu generieren als die Alternative der Wahl dar.

In WO 03/094774 "A dental or orthopaedic implant" von Plasma coatings limited wird das Verfahren der PEO (plasma electrolytic oxidation) als bekannter Prozess vorgestellt, der im Falle dentaler und orthopädischer Implantate verwendet wird. Dabei handelt es sich um calciumphosphathaltige Phasen in den oxidischen Konversionsschichten, die mit Wechselstrom (50 Hz - 60 Hz) im alkalischen Elektrolyten abgeschieden werden und bis zu Schichtdicken von 8 µm - 12 µm anwachsen. Dieser Prozess sorgt für einen verstärkten Einbau der calciumphosphathaltigen Phasen, um knochenähnliche Strukturen dem anwachsenden Zellmaterial zur Verfügung zu stellen und damit das Einheilverhalten zu verbessern. Diese Konversionsschicht ist durch die Prozessführung mit einer mangelhaften Oberflächenrauheit ausgestattet und die Porosität der Schicht kann nicht verhindern, dass evtl. Legierungsbestandteile des mechanisch beanspruchten Implantates durch die Schichten in den Körper diffundieren, Gewebereaktionen hervorrufen und zu einer kaskadenartigen Schädigung des Implantatlagers führen.

In WO 2005/084577 "Osseoinductive magnesiumtitanate implant and method for mamifacturing the same" von SUL wird ein Mehrstufenprozess beschrieben. So wird das zu beschichtende Implantat in eine magnesiumhaltige Lösung getaucht und mittels UV-Bestrahlung auf der Oberfläche verankert. Mit einer anschließenden anodischen Oxidation bei Spannungen von 60 V bis 500 V wird eine Schicht mit ungenügender Oberflächenrauheiten gebildet.

In WO 2005/087982 "Metal implants" wird in einem anodischen Prozess unter Verwendung von Phosphorsäure bei einer Spannung zwischen 50 V und 150 V eine farbige Schicht im Nanometerbereich erzeugt, die in einem nachfolgenden Prozess elektrochemisch mit Poren ausgestattet wird, in denen durch Einlagerung von bakterizidem Silber eine erhöhte antibakterielle Wirksamkeit bei endoprothetischen Implantaten erzielt wird.

In WO 2002/096475 "Modified Oxide" wird mit Hilfe der anodischen Oxidation in einem alkalischen Elektrolyten eine Doppelschichtstruktur mit einer tieferliegenden kompakten Barriereschicht und einer darauf aufbauenden porösen Oberschicht erzeugt. Die untere Schicht hat eine Schichtdicke zwischen 0.6 µm und 1.5 µm, während die obere Sicht eine Schichtdicke zwischen 0.1 µm und 0.5 µm besitzt. Das verfahrensbedingte Schichtsystem, das sich durch die angelegte Endspannung in einem Bereich zwischen 250 V - 500 V beeinflussen lässt, resultiert in einer Gesamtschichtdicke zwischen 0.8 µm und 1.5 µm. Die obere Schicht enthält neben Titan und Sauerstoff weiterhin ein Element (Calcium, Phosphor, oder Schwefel), das ebenfalls verfahrensbedingt aus dem Elektrolyt in die Schicht eingelagert wird. Die erhaltene Oberflächenrauheit entspricht dem Stand der Technik und wird durch keinen Verfahrensschritt optimiert.

In WO 9624391 A1 "Verfahren zur Herstellung einer gradierten Beschichtung aus Calciumphosphatphasen und Metalloxidphasen auf metallischen Implantaten" wird das Implantat als Substratelektrode in einem schwach sauren bis neutralen Dispersionselektrolyten, der ein Ca:P-Verhältnis ähnlich des handelsüblichen Hydroxylapatits besitzt, wechselweise bis zu einer Endspannung von 150 V potentiostatisch bzw. galvanostatisch kathodisch reduziert und potentiostatisch, potentiodynamisch oder galvanostatisch anodisch oxidiert. Somit wird eine wenig haftfeste Hydroxylapatitbeschichtung mit einer Dicke von ca. 0.5 µm erzeugt, die nicht verhindern kann dass Grundmaterial einer Titanlegierung in das umliegende Gewebe diffundieren kann.

In WO 2001/012883 A1 "Light alloy-based composite protective multifunctional coating" wird elektrochemisch auf der Basis der plasmachemischen anodischen Oxidation eine Oxidschicht gebildet, die durch Einlagerung weiterer Haupt- und Nebengruppenmetalle oder Verbindungen der IV. - VI. Hauptgruppe in die Poren charakterisiert ist. Eine mechanische Nachbehandlung ergibt eine Beschichtung, die Anforderungen wie Belastbarkeit, Härte und Widerstand gegen abrasive und korrosive Beanspruchungen gewachsen ist. Weiterhin wird Plastizität und Resistenz gegen dynamische Lastwechsel garantiert. Eine homogene Einlagerung von Calcium und eine optimierte Oberflächentopographie wird nicht erwähnt.

In US 2005/0031663 A1 "Implant Element" wird eine Oberflächenbeschichtung beschrieben, die durch eine optimierte Oberflächenbeschaffenheit (Rauheitsmittelwert = 40 nm), eine nachfolgende anodisch erzeugte 10 nm - 180 nm dicke Oxidschicht und mit mechanisch erzeugten Poren einer Breite von 1 µm - 10 µm ausgestattet ist.

In WO 200072776 A1 "Layer arranged on implant for bone or tissue stracture, such an implant, and a method for application of the layer" wird in Schwefelsäure durch die plasmachemische anodische Oxidation bis zu einer Endspannung von 400 V eine dicke Oxidschicht mit substantiellem Volumen an Poren erzeugt, um nachfolgend Wachstum stimulierende Faktoren einzubringen. Dabei wird auf die Oberflächenbeschaffenheit nur in dem Sinne eingegangen, dass die Unebenheiten der Oberfläche durch HF abgeätzt werden.

In WO 2002/078759 A1 "Bioaktive Oberflächenschicht, insbesondere für medizinische Implantate und Prothesen" wird mittels der plasmachemischen anodischen Oxidation in einem basischen Elektrolyten der Calcium- und Phosphationen in einem weiten Verhältnisbereich enthält eine poröse, wasserlösliche Schicht gebildet. Das Ca: P Verhältnis der Schicht soll unter anderem dem Knochenwachstum fördernden Hydroxylapatit entsprechen. Weiterhin dienen die Poren als Wirkstoffdepots für entzündungshemmende und Wachstum stimulierende Medikamente. Optimierte Oberflächentopographien und Loslösemomente für die evtl. Rückholbarkeit der Implantate findet sich nicht berücksichtigt.

In WO 2007/090433 A2 "Purified Oxides with novel morphologies formed from Ti-alloys" wird in einem sauren Elektrolyten aus Schwefel- und Phosphorsäure aber auch in basischen Elektrolyten mit Calcium- und Phosphorverbindungen bis zu einer Endspannung von 150 V - 250 V plasmachemisch anodisch oxidiert. Die Kombination aus Strahlbehandlung mit Sand und Beizen vor der Beschichtung führt zu Endrauheiten (Rₐ) im Bereich zwischen 3.4 µm und 4.4 µm.

In WO 2007/073213 A1 "Micro arc assisted lectroless electroplating methods" wird eine Methode vorgestellt wie auf Leichtmetallen im Anschluss an eine plasmachemische anodische Oxidation eine stromlose Beschichtung am Beispiel des Nickels erfolgen kann und Magnesium so vor Korrosion schützt.

In CN 1974876A wird eine 3 min. - 60 min. dauernde Beschichtung gezeigt, bei der Al₂O₃, SiC und TiO₂ in eine 25 µm - 250 µm dicke Schicht eingelagert werden können.

In EP 1 527 790 A1 "Implantat mit einer keramischen Beschichtung" wird zwischen einer Weichgewebe Kontaktfläche und einer Knochenkontaktfläche unterschieden. Die Weichgewebekontaktfläche wird durch eine keramische Beschichtung funktionalisiert. Diese Funktionalisierung erfolgt durch die anodische Oxidation unter Funkenentladung.

In EP 462 073 B1 "Elektrolyt zur Erzeugung dünner, schwarzer Konversionsschichten auf Leichtmetallen" wird mit Hilfe der anodischen Oxidation unter Funkenentladung ein Elektrolyt vorgestellt, mit dem die in der Patentschrift charakterisierten Eigenschaften zu erreichen sind. Dabei stehen keine Anforderungen im Bereich der Osteosynthese im Vordergrund, sondern technisch orientierte Anforderungen im Bereich optischer Bauteile.

In JP 08289927A_TR "Implant in bone and ist manufacture" wird die Methode des Shot Peenings (Kugelstrahlen) vorgestellt und an unbeschichteten Titanoberflächen zum Einsatz gebracht, die Oberflächeneigenschaften damit verbessert und die darin erwähnten und bewiesenen Verbesserungen für die Osteosynthese erzielt. Die keramischen Kugeln liegen in einem Durchmesserbereich von 50 µm -500 µm.

In DE 197 50 502 A1 "Verfahren zur Erhöhung der Lebensdauer von zahntechnischen Teilen" wird die Methode des Kugelstrahlens vorgestellt, um die Ermüdungsfestigkeit von metallischen Implantaten bei schwingenden Belastungen zu verbessern. Durch das Kugelstrahlen werden Druckspannungen in das Metall eingebracht. Die Stahl- oder Glaskugeln liegen in einem Durchmesserbereich von 400 µm - 600 µm.

In US 2004/0158330 A1 "Surface treated metallic implant and blasting material" wird auf eine gesinterte, kristalline und bioaktive Oberflächenbeschichtung eines Metallimplantats durch eine Strahlbehandlung eine erhöhte chemische Stabilität und zusätzlich eine erhöhte Rauheit erreicht.

In DE 10 2004 008 687 A1 "Prothese aus Titan oder Titanlegierungen und Verfahren zur Oberflächenbehandlung einer solchen Prothese" wird eine Methode der Strahlbehandlung mit Trockeneis vorgestellt. Die Oberfläche einer Hüftgelenkprothese wird mit Trockeneispellets mit einem Durchmesser von 2 mm bestrahlt. Die Oberfläche weist dann eine definierte Oberflächenrauheit auf, d.h. Risskeime die zu Spannungsrissen oder zu Bruch fuhren könnten, werden verschlossen, stärkere Unrauhigkeiten werden beseitigt und eine Mikrorauhigkeit wird geschaffen, die eine optimale Festigkeit des Implantats im Knochen gewährleistet.

In DE 195 17 275 C2_EQ "Verfahren zur Herstellung einer Prothese aus Titan bzw. Titanlegierungen und nach dem Verfahren hergestellte Prothese" wird eine Prothese vorgestellt, die wenigstens an den mechanisch beanspruchten Oberflächen eines Implantates durch Kugelstrahlen mit Stahlkugeln behandelt ist und im Anschluss mit Glaskugelbestrahlung fertig gestellt wird.

In DE 41 26 800 A1 "Verbundwerkstoff auf Metallbasis" wird ein Verbundwerkstoff vorgestellt mit einer homogenen 1 µm dicken Schicht eines glasigen, glaskeramischen und/oder keramischen Materials als Ausgangssubstrat mit einer evtl. durch Haftvermittler fixierten Schicht aus Kunststoff, Keramik oder Zement darüber. Im Anschluss wird auf die Oberflächenschicht Strahlgut aus der Gruppe Apatit, Wollastonit ,Ca₇Mg₂P₆O₂₄ sowie Nebenkristallphasen aus der Gruppe Cristobalit, Diopsit und Forsterit mit einem Durchmesser zwischen 50 µm und 250 µm in die Schicht eingearbeitet. Die Eindringtiefe beträgt dabei bis zu 1 µm.

In EP 827722 A2 "Verfahren zur Erhöhung der Lebensdauer von zahntechnischen Teilen" wird ein Verfahren vorgestellt, in dem durch Kugelstrahlen mit Kugel in einem Durchmesserbereich zwischen 420 µm und 590 µm Druckspannungen in die Oberflächenschicht eines Implantats aus dem Dentalbereich eingebracht werden und damit die Lebensdauer des Implantats erhöht wird.

In EP 602712 B1 "Method of surface finishing orthopaedic implant devices using a bioactive blasting medium" wird ein Verfahren vorgestellt mit dem durch Strahlbehandlung mittels eines bioaktiven Strahlmediums, das aus einem bioaktiven Granulat gebildet ist, innerhalb des Körpers nicht bioinert ist und für das umgebende Körpergewebe unschädlich ist die Biokompatibilität erhöht wird. Das Strahlmittel ist aus der Gruppe ausgewählt, die aus Calciumphosphatkeramiken, Phosphatglas-Zusammensetzungen, biologisch absorbierbarem Glas, teilweise absorbierbarem Glas, vollständig biologisch absorbierbaren Biokeramiken, teilweise biologisch absorbierbaren Biokeramiken und nicht absorbierbaren Biokeramiken besteht.

In US 5,057,108 A "Method of surface finishing orthopedic implant devices" wird ähnlich zum Patent EP602712B1 "Method of surface finishing orthopaedic implant devices using a bioactive blasting medium" ein Strahlverfahren vorgestellt, bei dem zunächst eine Behandlung mit Stahlkugeln, anschließender Behandlung mit Glaskugeln und abschließender elektrochemischer Politur ein optimiertes Implantat aus Edelstahl bereitgestellt wird.

### Aufgabenstellung und Erfindungsbeschreibung

Der vorliegenden Erfindung lag die Aufgabe zugrunde ein Verfahren zu entwickeln, das im elektrochemischen Tauchverfahren biologisch degradationsstabile Oberflächenschichten auf Titan oder Titanbasislegierungen erzeugt, die Oberflächenschichten durch nachfolgende Modifizierungen und Optimierungen bei gleichzeitig beibehaltener Degradationsstabilität einstellbare Eigenschaften besitzen und im Ergebnis verbessertes Osteosynthesematerial bereitgestellt werden kann.

Einstellbare Eigenschaften der Oberflächenschicht nach einer Kombination aus einem elektrochemischen Tauchverfahren und darauf abgestimmter Nachbehandlung lauten zum Beispiel: bioinert, bioaktiv, chemisch inert, chemisch aktiv, diffusionshemmend, degradationsstabil, porenreich, porenarm, haftfest, sterilisierbar, hitzebeständig, geringe Rauheit, reduzierte Kaltschweißneigung, duktil, knochenähnlich, spröde, etc.

Eine wichtige Kombination von Eigenschaften dieser Oberflächenbeschichtung betrifft die fakultative Entfernbarkeit eines Implantates nach einer komplikationslosen Frakturheilung, wenn das Osteosynthesematerial ausgedient hat und entfernt werden kann. Die Aufgabe wird erfindungsgemäß dadurch gelöst, dass eine geeignete Vorbehandlung nach dem Fertigungsprozess möglichst reproduzierbare und optimierte Ausgangsoberflächen bereitstellt. Im Anschluss wird eine poröse und plastisch verformbare Oberflächenbeschichtung unter Anwendung des elektrochemischen Tauchverfahrens in einem erfindungsgemäßen Elektrolyten und dessen charakteristischem Schichtbildungspotentialbereich aufgebracht und anschließend durch eine darauf abgestimmte Nachbehandlung mit Strahlverfahren eine einstellbare Oberflächentopographie mit spezifischer Rauheit bereitgestellt. Die erfindungsgemäße Oberflächenbeschichtung hat die Eigenschaft, dass sie sich durch weitgehende Stabilität gegenüber Solvatations-, Diffusions- und Zersetzungsvorgängen in biologischen Systemen sowie gegenüber thermischen Belastungszyklen, wie sie bei der thermischen Dampfsterilisation in Autoklaven zum Einsatz kommen, auszeichnet und dabei die Oberflächenbeschichtung über mehrere Monate ihre Degradationsresistenz und Haftfestigkeit beibehält. Damit können die Implantate nach der Heilungsphase problemlos wahlweise im Körper verbleiben oder entfernt werden. Durch ein geeignetes Elementverhältnis ist es weiterhin möglich die Oberflächenbeschichtung auch bioaktiv zu gestalten und somit den Besiedelungsfortschritt durch Osteoblasten zu beschleunigen. Damit kann ein Teilerfolg gegenüber der konkurrierenden Besiedelung durch Bakterien und einer nachfolgenden Degradation des Implantatlagers durch bakterielle Inflammation erzielt werden. Diese erhöhten Anforderungen ergeben sich vor allem aus der weitaus größeren Komplikationsrate bei offenen Frakturen. In diesem Fall kann ein Restporenvolumen der Oberflächenbeschichtung mit geeigneten bakteriziden Wirkstoffen beladen werden oder durch weitere bakterizide Oberflächenbeschichtungen resistent gegenüber bakteriellen Inflammationen ausgestattet werden.

### Ausführungsbeispiel

Ein wässriger Elektrolyt der 0.05 mol·l⁻¹ - 0.5 mol·l⁻¹ Ethylendiamintetraessigsäure Calcium-di-Natriumsalz Dihydrat (C₁₀H₁₂N₂O₈CaNa₂ · 2 H₂O), 0.05 mol·l⁻¹ - 0.5 mol·l⁻¹ Ammoniumdihydrogenphosphat (NH₄H₂PO₄), 0.5 mol·l⁻¹ - 5 mol·l⁻¹ Wasserstoffperoxid (H₂O₂) und 0.003 mol·l⁻¹ -0.03 mol·l⁻¹ Pyridin-2,6-dicarbonsäure (C₇H₅O₄N) enthält, wird auf einen pH-Wert von 5 - 7 eingestellt. Reintitan bzw. eine Titanlegierung (≥ 60 M.-% Titan) mit einer Ausgangsrauheit von Rₐ ≤ 2.5 µm oder Rₜ ≤ 15 µm wird im Ultraschallbad gereinigt und im wässrigen Elektrolyten teilweise oder vollständig untergetaucht und anodisch gepolt. Das elektrochemische Tauchverfahren erfolgt bei Gleichspannung oder gepulster Gleichspannung (f ≤ 1500 s⁻¹) und einer Stromdichte von ≤ 15 A·dm⁻² bei einer Elektrolyttemperatur von 15 °C - 50 °C unter Atmosphärendruck. Nach Erreichen der Endspannung von ≤ 400 V wird der beschichtete Körper in einer mehrstufigen Spülung mit Wasser von anhaftendem Elektrolyten befreit und mit Luft getrocknet. Anschließend wird der beschichtete Körper einer Strahlbehandlung unterzogen bei der das Porenvolumen der erzeugten Oberflächenschicht durch mechanische Verdichtung von ursprünglich 30 Vol.-% - 70 Vol.- % auf 0 Vol.-% - 30 Vol.-% verringert wird. Dazu werden pneumatisch beschleunigte Strahlkörper aus Glas mit einem Durchmesser von 5 µm - 200 µm verwendet.

## Patentansprüche

1. Elektrochemisches Tauchverfahren in einem wässrigen Elektrolyt zur Erzeugung einer biologisch degradationsstabilen Oberflächenschicht auf Grundkörpern aus Titan oder Titanbasislegierungen mit anodischer Polung der Grundkörper,
**gekennzeichnet dadurch, dass**
der Elektrolyt als wässrige Lösung mit den erfindungsgemäßen Bestandteilen 0,05 - 0,5 mol/l Calcium-di-Natrium-Ethylendiamintetraessigsäure (C₁₀H₁₂N₂O₈CaNa₂), 0,05 - 0,5 mol/l Ammoniumdihydrogenphosphat (NH₄H₂PO₄), 0,5 - 5,0 mol/l Wasserstoffperoxid (H₂O₂) und 0,003 - 0,03 mol/l Pyridin-2,6-dicarbonsäure (C₇H₅O₄N) gebildet wird.

2. Verfahren nach Anspruch 1,
gekennzeichnetdadurch, dass
der pH-Wert der wässrigen Elektrolytlösung gleich oder größer 5 und gleich oder kleiner 7 eingestellt wird.

3. Verfahren nach den Ansprüchen 1 und 2,
**gekennzeichnet dadurch, dass**
unter Normaldruck die Temperatur des Elektrolyten zwischen 15 °C und 50 °C eingestellt wird.

4. Verfahren nach den Ansprüchen 1 bis 3,
**gekennzeichnet dadurch, dass**
der Grundkörper aus technisch reinem Titan oder einer Titanlegierung mit mindestens 60 Masse-% Titan besteht.

5. Verfahren nach den Ansprüchen 1 bis 4,
**gekennzeichnet dadurch, dass**
die Oberflächenrauheit des Titangrundkörpers vor der elektrolytischen Behandlung Rₐ kleiner 2,5 µm und Rₜ kleiner 15 µm definiert erzeugt wird.

6. Verfahren nach den Ansprüchen 1 bis 5,
**gekennzeichnet dadurch, dass**
der Grundkörper teilweise oder vollständig in den Elektrolyt eingetaucht, anodisch gepolt und mit Gleichspannung kleiner oder gleich 400 V elektrochemischen Reaktionen an der Oberfläche unterworfen wird.

7. Verfahren nach den Ansprüchen 1 bis 6,
**gekennzeichnet dadurch, dass**
die Gleichspannung periodisch geändert wird.

8. Verfahren nach den Ansprüchen 1 bis 7,
**gekennzeichnet dadurch, dass**
innerhalb einer Periode das Zeitintervall bei hoher und niedriger Spannung annähernd gleich eingestellt wird.

9. Verfahren nach den Ansprüchen 1 bis 8,
**gekennzeichnet dadurch, dass**
die Frequenz der periodischen Spannungsänderung gleich oder kleiner 1500 s⁻¹ eingestellt wird.

10. Verfahren nach den Ansprüchen 1 bis 9,
**gekennzeichnet dadurch, dass**
der Titangrundkörper und die erfindungsgemäß erzeugte Oberflächen schicht nach der anodischen elektrolytischen Behandlung vorzugsweise mit Wasser von den anhaftenden Resten des Elektrolyts gereinigt und anschließend mit Luft getrocknet wird.

11. Verfahren nach den Ansprüchen 1 bis 10,
**gekennzeichnet dadurch, dass**
die Reinigungder Titangrundkörper nach der anodischen elektrolytischen Behandlung vorzugsweise mit Wasser von den anhaftenden Resten des Elektrolyts mehrstufig erfolgt.

12. Verfahren nach den Ansprüchen 1 bis 11,
**gekennzeichnet dadurch, dass**
das Porenvolumen in der erzeugten Oberflächenschicht durch mechanische Verdichtung von ursprünglich 30 Vol.-% bis 70 Vol.-% auf 0 Vol.-% bis 30 Vol.-% verringert wird.

13. Verfahren nach den Ansprüchen 1 bis 12,
**gekennzeichnet dadurch, dass**
die Verdichtung durch das Aufprallen von pneumatisch beschleunigten Strahlkörpern erfolgt.

14. Verfahren nach den Ansprüchen 1 bis 13,
**gekennzeichnet dadurch, dass**
die Strahlkörper aus Glasperlen mit einem Durchmesser von 5 µm bis 200 µm bestehen.

## Claims

1. Electrochemical immersion method in an aqueous electrolyte for producing a biologically degradation-stable surface layer on base bodies made of titanium or titanium-based alloys, with anodic polarization of the base bodies,
**characterized in that**
the electrolyte is formed as an aqueous solution with the inventive components 0,05 - 0,5 mol/l calcium disodium ethylenediaminetetraacetic acid (C₁₀H₁₂N₂O₈CaNa₂), 0.05 - 0.5 mol/I ammonium dihydrogen phosphate (NH₄H₂PO₄), 0.5 - 5.0 mol/l hydrogen peroxide (H₂O₂), and 0.003 - 0.03 mol/I pyridine-2.6-dicarboxylic acid (C₇H₅O₄N).

2. Method according to claim 1,
**characterized in that**
the pH-value of the aqueous electrolyte solution is adjusted to be equal to or greater than 5 and equal to or smaller than 7.

3. Method according to claims 1 and 2,
**characterized in that**
under normal pressure the temperature of the electrolyte is set between 15°C and 50°C.

4. Method according to claims 1 to 3,
**characterized in that**
the base body is formed of technically pure titanium or a titanium alloy with at least 60 % by mass of titanium.

5. Method according to claims 1 to 4,
**characterized in that**
the surface roughness of the titanium base body prior to the electrolytic treatment Rₐ smaller than 2.5 µm and Rₜ smaller than 15 µm is produced in a defined manner.

6. Method according to claims 1 to 5,
**characterized in that**
the base body is partially or wholly immersed in the electrolyte, anodically polarized, and is subjected to electrochemical reactions on the surface at a direct voltage smaller than or equal to 400 V.

7. Method according to claims 1 to 6,
**characterized in that**
the direct voltage is periodically varied.

8. Method according to claims 1 to 7,
**characterized in that**
within one period the time interval at high and low voltage is adjusted to be approximately equal.

9. Method according to claims 1 to 8,
**characterized in that**
the frequency of the periodic voltage variation is set to be equal to or smaller than 1500 s⁻¹.

10. Method according to claims 1 to 9,
**characterized in that**
after the anodic electrolytic treatment the titanium base body and the inventively produced surface layer is preferably cleaned with water to remove the adhering electrolyte residues and dried with air subsequently.

11. Method according to claims 1 to 10,
**characterized in that**
after the anodic electrolytic treatment the cleaning of the titanium base bodies preferably with water to remove the adhering electrolyte residues is carried out in multiple stages.

12. Method according to claims 1 to 11,
**characterized in that**
the pore volume in the produced surface layer is reduced by mechanical compression from originally 30 % by volume - 70 % by volume to 0 % by volume - 30 % by volume.

13. Method according to claims 1 to 12,
**characterized in that**
the compression is accomplished by the impingement of pneumatically accelerated blast bodies.

14. Method according to claims 1 to 13,
**characterized in that**
the blast bodies are formed of glass beads having a diameter of 5 µm to 200 µm.

## Revendications

1. Procédé d'immersion électrochimique dans un électrolyte aqueux pour produire une couche de surface biologiquement stable vis-à-vis de la dégradation sur des corps de base en titane ou en alliages à base de titane avec polarisations anodique du corps de base,
**caractérisé en ce que** le
l'électrolyte est formé comme une solution aqueuse présentant, selon l'invention, les éléments constitutifs suivants :
0,05 - 0,5 mol/l de calcium-di-sodium-éthylène diamine acide tétraacétique (C₁₀H₁₂N₂O₈CaNa₂)
0,05 - 0,5 mol/l de dihydrogénophosphate d'ammonium (NH₄H₂PO₄)
0,5 - 5,0 mol/l de peroxyde d'hydrogène (H₂O₂), et
0,003 - 0,03 mol/l de pyridine-2,6-acide dicarboxylique (C₇H₅O₄N).

2. Procédé selon la revendication 1,
**caractérisé en ce que** la valeur du pH de la solution électrolytique aqueuse est réglée égale ou supérieure à 5, et égale ou inférieure à 7.

3. Procédé selon les revendications 1 et 2,
**caractérisé en ce que** la température de l'électrolyte est réglée, sous pression normale, entre 15° C et 50° C.

4. Procédé selon les revendications 1 à 3,
**caractérisé en ce que** le corps de base est composé de titane techniquement pur ou d'un alliage de titane avec au moins 60 % de titane en masse.

5. Procédé selon les revendications 1 à 4,
**caractérisé en ce que** la rugosité de surface du corps de titane est engendrée de manière définie, avant le traitement électrolytique, à une valeur Ra inférieure à 2,5 µm et Rt inférieure à 15 µm.

6. Procédé selon les revendications 1 à 5,
**caractérisé en ce que** le corps de base est immergé partiellement ou totalement dans l'électrolyte, avec polarisation anodique et est soumis à des réactions électrochimiques à la surface avec une tension continue inférieure ou égale à 400 V.

7. Procédé selon les revendications 1 à 6,
**caractérisé en ce que** la tension continue est modifiée périodiquement.

8. Procédé selon les revendications 1 à 7,
**caractérisé en ce qu'**à l'intérieur d'une période l'intervalle temporel est choisi de façon approximativement égale pour une tension élevée et pour une tension faible.

9. Procédé selon les revendications 1 à 8,
**caractérisé en ce que** la fréquence de la modification périodique de tension est réglée à une valeur égale ou inférieure à 1500 s⁻¹.

10. Procédé selon les revendications 1 à 9,
**caractérisé en ce que** le corps de base en titane et la couche de surface engendrée selon l'invention sont nettoyés après le traitement électrolytique anodique de préférence avec de l'eau pour supprimer les restes d'électrolyte adhérent et ensuite séchés à l'air.

11. Procédé selon les revendications 1 à 10,
**caractérisé en ce que** le nettoyage du corps de base en titane après le traitement électrolytique anodique pour supprimer les restes d'électrolyte adhérent avec de l'eau a de préférence lieu en plusieurs passes.

12. Procédé selon les revendications 1 à 11,
**caractérisé en ce que** le volume des pores dans la couche de surface engendrée est réduit par compactage mécanique d'une valeur initiale de 30 % à 70 % en volume jusqu'à 0 % à 30 % en volume.

13. Procédé selon les revendications 1 à 12,
**caractérisé en ce que** le compactage a lieu par impact avec des corps projectiles accélérés par voie pneumatique.

14. Procédé selon les revendications 1 à 13,
**caractérisé en ce que** les corps projectiles sont des perles de verre avec un diamètre de 5 µm à 200 µm.
